(19) Europäisches Patentamt / European Patent Office / Office européen des brevets

(11) **EP 4 280 400 A1**

(12) **EUROPEAN PATENT APPLICATION**
published in accordance with Art. 153(4) EPC

(43) Date of publication:
**22.11.2023 Bulletin 2023/47**

(51) International Patent Classification (IPC):
*H01S 5/183* (2006.01)        *H01S 5/022* (2021.01)
*H01S 5/024* (2006.01)

(21) Application number: **22739193.5**

(22) Date of filing: **18.01.2022**

(86) International application number:
**PCT/CN2022/072597**

(87) International publication number:
**WO 2022/152318 (21.07.2022 Gazette 2022/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.01.2021   CN 202110059453**

(71) Applicant: **Beijing Laserconn Tech Co., Ltd.**
**Beijing 100192 (CN)**

(72) Inventor: **LI, Yang**
**Beijing 100085 (CN)**

(74) Representative: **Wang, Bo**
**Panovision IP**
**Ebersberger Straße 3**
**85570 Markt Schwaben (DE)**

(54) **LASER BASED ON VCSEL IMAGING AND COAXIAL VISUALIZATION DESIGN**

(57)     Disclosed in the present invention is a laser based on VCSEL imaging and coaxial visualization design, comprising a VCSEL planar array light source, a heat sink, an imaging lens, and a planar mirror which are provided in a housing. The VCSEL planar array light source is provided on the heat sink and is used for emitting VCSEL laser light; the imaging lens and a primary optical axis of the VCSEL planar array light source are coaxially provided; the planar mirror and the primary optical axis of the VCSEL planar array light source are provided at an angle $\alpha$, such that the laser light is deflected, and a real image is formed on a primary optical axis of the deflected VCSEL laser; an optical window is provided at an image position of the deflected VCSEL laser light; and an observation position is provided on the side of the planar mirror away from the imaging lens, and is coaxially provided with an optical center axis of the deflected laser light. In the laser, the transmission process of the VCSEL laser light is controlled by using an optical imaging principle, and the VCSEL laser light is deflected by providing the planar mirror behind the imaging lens, such that an observation position is formed on the side of the planar mirror away from a real image point to realize coaxial observation.

FIG. 1

EP 4 280 400 A1

**Description**

**BACKGROUND**

**Technical Field**

The present invention relates to a laser based on VCSEL imaging and coaxial visualization design.

**Related Art**

Lasers have a wide range of uses in the fields of industrial laser processing and medical lasers. In the process of operating with the lasers, especially when using the lasers for medical treatment, CCD cameras are required for field-of-view observation and auxiliary visual localization to facilitate observation and adjustment of the lasers.
At present, field-of-view observation and visual localization devices are usually provided separately from laser therapeutic instrument handpieces, and imaging light is non-coaxial with a laser beam, making it impossible to directly observe the field of view of therapy. Moreover, it needs complicated coordinate conversion for the control of the lasers.

**SUMMARY**

The technical problem to be solved by the present invention is to provide a laser based on VCSEL imaging and coaxial visualization design.
In order to achieve the technical objective above, the present invention adopts the following technical solution: A laser based on VCSEL imaging and coaxial visualization design, including

a housing, defining a hollow holding cavity;
a VCSEL planar array light source and a heat sink, provided in the housing, the VCSEL planar array light source being provided on the heat sink for emitting a VCSEL laser light;
an imaging lens, provided in the housing, the imaging lens being provided coaxially with a main optical axis of the VCSEL planar array light source for imaging the VCSEL laser light, and a distance between the VCSEL planar array light source and the imaging lens being greater than a focal length of the imaging lens;
a planar reflector, provided in the housing, the planar reflector being located behind the imaging lens and provided at an angle of $\alpha$ with the main optical axis of the VCSEL planar array light source, so as to cause deflection of the laser light passing through the imaging lens and to form a real image in a main optical axis of the deflected VCSEL laser light, with $0° < \alpha < 90°$, and the planar reflector being configured to provide total reflection of the VCSEL laser light and to allow transmission of other wavelengths of lights;
an optical window, provided on the housing and at a position of a real image point of the deflected VCSEL laser light; and
an observation position, provided on a side of the planar reflector away from the imaging lens and the optical window, the observation position being provided coaxially with an optical center axis of the deflected VCSEL laser light.

Preferably, a distance c between the VCSEL planar array light source and the imaging lens, a distance a between the imaging lens and the planar reflector, and a distance b between the planar reflector and the optical window satisfy the following equation: $1/(a + b) + 1/c = 1/f$, where f represents the focal length of the imaging lens.
Preferably, a diameter D of the imaging lens satisfies the following equation: $D \geq m + 2c \times \tan(\theta/2)$, where m is a spot diameter of the VCSEL planar array light source and $\theta$ is a divergence full angle of the VCSEL planar array light source.
Preferably, the imaging lens is a biconvex lens.
Preferably, a surface of the planar reflector facing the imaging lens is provided with a highly reflective coating for reflecting the VCSEL laser light.
Preferably, the planar reflector is provided at an angle of 45 degrees with the main optical axis of the VCSEL planar array light source.
Preferably, a CCD imaging device is provided at the observation position.
Preferably, an observation window is provided at the observation position, or a combination of an observation window and a magnifying glass is provided at the observation position.
Preferably, the laser further includes a semiconductor chilling plate and a heat pipe, where a cold end of the semiconductor chilling plate is configured to cool the optical window by the housing, and a hot end of the semiconductor chilling plate is configured to dissipate heat through the heat pipe and the heat sink.
Preferably, the laser further includes a fan and a control unit, the fan being provided below the heat sink for dissipating heat from the heat sink, and the semiconductor chilling plate, the VCSEL planar array light source and the fan being electrically connected to the control unit, respectively.

The laser provided by the present invention utilizes the optical imaging principle to control the transmission process of the VCSEL laser. By providing the planar reflector between the imaging lens and the image point of the undeflected laser light, the VCSEL laser light is deflected, and the observation position is formed on the side of the planar reflector away from the real image point, thus realizing coaxial observation. The laser above may be used as a laser therapeutic instrument and may also be applied in the field of laser processing.

**BRIEF DESCRIPTION OF THE DRAWINGS**

FIG. 1 is a schematic structural diagram of a laser provided by the present invention.
FIG. 2 is a schematic diagram showing an image-forming principle of the laser provided by the present invention.
FIG. 3 is a schematic diagram showing positional relationship of components in an optical system in the laser provided by the present invention.
FIG. 4 is a schematic diagram showing dimensional relationship of an imaging lens, a planar reflector and a virtual image in the laser provided by the present invention.

**DETAILED DESCRIPTION**

The technical solutions of the present invention will be further described in detail below in conjunction with the drawings and specific embodiments.

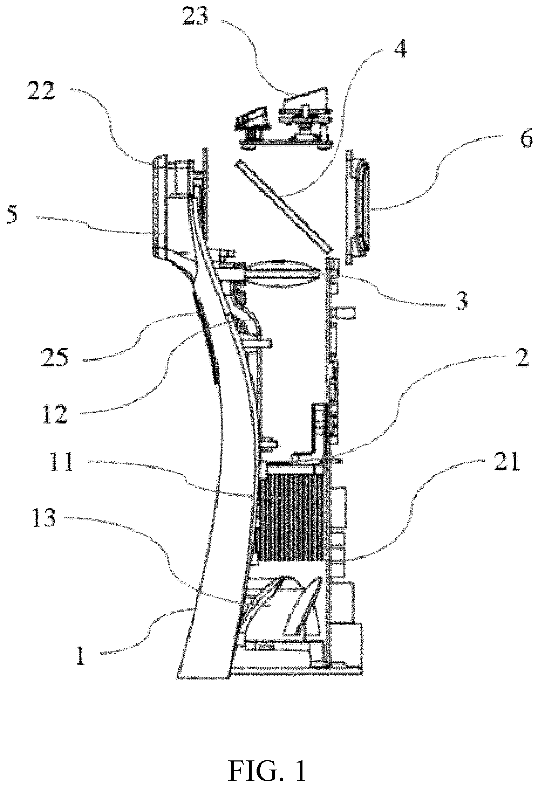

As shown in FIG. 1, a laser based on VCSEL (vertical cavity surface emitting laser) imaging and coaxial visualization design provided by an embodiment of the present invention includes a housing 1 defining a hollow holding cavity, and further includes a VCSEL planar array light source 2, an imaging lens 3, and a planar reflector 4 provided in the housing 1.

The housing 1 is of elongated. The housing 1 is configured to connect and fix internal devices, and adopts ergonomic design to increase the grip comfort while playing a certain role in protecting the internal precision component of products.

The VCSEL planar array light source 2 is provided in the housing 1 near the lower portion (see the orientation shown in FIG. 1). The VCSEL planar array light source 2 is provided on the heat sink 11 and is configured to emit a VCSEL laser light upwards. The VCSEL planar array light source 2 is implemented by a plurality of VCSEL laser chips. The VCSEL laser chips are characterized by long life, flexible packaging structure, and high long-term reliability. There may be one or more VCSEL laser chips, and the wavelength is selectable or chips at a plurality of wavelengths may be combined for use. The VCSEL planar array light source with regular and dense light-emitting diodes is a planar light source with uniform light-emitting property. Divergence angles in all directions of VCSEL are the same and not large (full angle of about 12 to 20 degrees). Therefore, it is convenient to use a convex lens for imaging, and a flat-topped spot (i.e., uniform spot, the same below) with excellent uniformity can be obtained at a position of an image point, thereby serving various industrial, medical and scientific research applications.

An imaging lens 3 and a planar reflector 4 are provided on a main optical axis 20 of the VCSEL laser light. The imaging lens 3 is coaxial with a main optical axis of a VCSEL planar array light source 2 for focusing imaging of the VCSEL laser light. The imaging lens 3 uses a biconvex lens. The imaging lens 3 is provided in the housing 1 by a fixing bracket. The imaging lens 3 uses the biconvex lens for imaging. The planar reflector 4 is located behind the imaging lens 3 (i.e., the upper portion as shown in FIG. 1) and is provided at an angle of $\alpha$ with the main optical axis 20 of the VCSEL planar array light source 2, so as to cause deflection of the laser light passing through the imaging lens 3 (as being deflected to the left shown in FIG. 1) and to form a real image 2B in a main optical axis 20' of the deflected VCSEL laser light, with $0° < \alpha < 90°$. The planar reflector 4 is configured to provide total reflection of the VCSEL laser light and to allow transmission of other wavelengths of lights. A surface of the planar reflector 4 may be coated with a highly reflective coating according to the wavelength of the laser chip for enhancing the reflectivity of the planar reflector 4 for laser lights within a predetermined wavelength range.

An optical window 5 is provided on the housing 1. The optical window 5 is provided at the position of an image point (i.e., the position where the real image 2B is located) after the VCSEL laser light is deflected. A laser beam passes through the optical window 5 and is then imaged on a surface (i.e., a treatment surface or a working surface) of an object in contact with the optical window 5. The optical window 5 may use an optical device that has light transmittance performance and a cooling effect such as sapphire.

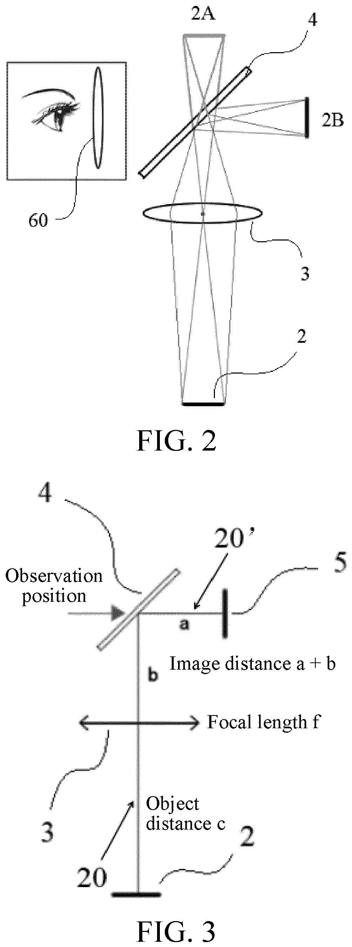

As shown in FIG. 2 and FIG. 3, the imaging lens 3 has a focal length f, an object distance c, and an image distance a + b in line with the imaging principle: $1/(a + b) + 1/c = 1/f$, where c is a distance between the VCSEL planar array light source 2 and the imaging lens 3, a is a distance between the imaging lens 3 and the planar reflector 4, and b is a distance between the planar reflector 4 and the optical window 5.

In order to obtain the real image of the VCSEL laser light, the distance c between the VCSEL planar array light source 2 and the imaging lens 3 should be greater than the focal length f of the imaging lens 3. Preferably, the VCSEL planar array light source 2 is provided at a position of 2× focal length of the imaging lens 3.

The imaging of the VCSEL planar array light source 2 can be zoomed in or out by adjusting the object distance c and the image distance a + b. The imaging magnification = m'/m = (a + b)/c. At a typical 1:1 imaging magnification, a + b = c = 2f. In the present invention, m represents a diameter of the uniform spot generated by the VCSEL planar array light source 2, and m' represents a diameter of the real image 2.

The dimensions of the imaging lens 3 are required to cover the entire range of the VCSEL laser beam to avoid optical energy leakage. That is, a diameter D of the imaging lens 3 satisfies the following equation: $D \geq m + 2c \times \tan(\theta/2)$, where $\theta$ is a divergence full angle of the VCSEL planar array light source 2.

As shown in FIG. 2, the planar reflector 4 may be inserted at any position between the imaging lens 3 and a position of a virtual image point 2A of the undeflected laser light. The position of the virtual image point 2A is an imaging position at which the VCSEL laser beam is not deflected. The deflected laser light forms a real image at the position of a real image point 2B (i.e., where the optical window 5 is located). The real image is rotated with respect to the virtual image, and the real image and the virtual image are equal in size. The dimensions of the planar reflector 4 are required to cover the entire range of the VCSEL beam to avoid optical energy leakage.

The following is an exemplary description of the process of calculating a diameter D' of the planar reflector 4 when the imaging lens 3 forms an equal-sized real image. When the imaging lens 3 forms an equal-sized real image, m' = m and a + b = c = 2f.

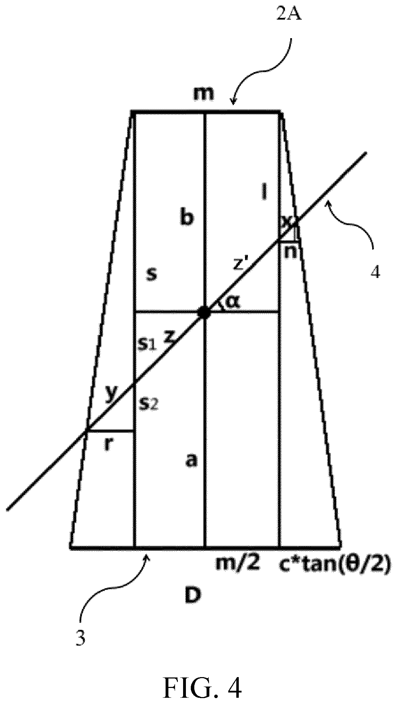

As shown in FIG. 4, the diameter D' of the planar reflector 4 satisfies the following equation:

$$D' \geq x+y+z+z' \quad\quad (1)$$

where

$$z = z' = m/2\cos(\alpha); \quad\quad (2)$$

the equation for x and y is given below:
it can be seen from FIG. 4,

$$\frac{a+b}{c*\tan(\theta/2)} = \frac{l}{n} = \frac{s}{r} \quad ; \quad\quad (3)$$

$$l = b - m *\tan(\alpha)/2; \quad\quad (4)$$

$$n = x*\cos(\alpha) + x*\sin(\alpha)/\tan(\theta); \quad\quad (5)$$

$$\frac{a+b}{c*\tan(\theta/2)} = \frac{b - m*\tan(\alpha)/2}{x*(\sin(\alpha)/\tan(\theta)+\cos(\alpha))} \quad ; \quad\quad (6)$$

calculated by equation (6):

$$x = \frac{b*c*\tan(\theta/2)-m*c*\tan(\alpha)*\tan(\theta/2)/2}{(a+b)*(\sin(\alpha)/\tan(\theta)+\cos(\alpha))} \quad ; \quad\quad (7)$$

$$s = b+s1+s2; \qquad (8)$$

$$s1 = m*\tan(\alpha)/2; \qquad (9)$$

$$s2 = y*\sin(\alpha); \qquad (10)$$

$$r = y*\cos(\alpha); \qquad (11)$$

$$\frac{a+b}{c*\tan(\theta/2)} = \frac{b + m*\tan(\alpha)/2 + y*\sin(\alpha)}{y*\cos(\alpha)} ; \qquad (12)$$

calculated by equation (12):

$$y = \frac{b*c*\tan(\theta/2)+m*c*\tan(\alpha)*\tan(\theta/2)/2}{(a+b)*\cos(\alpha)-c*\sin(\alpha)*\tan(\theta/2)} . \qquad (13)$$

The minimum value of D' can be derived from equation (1), equation (2), equation (7) and equation (13).

Preferably, the planar reflector 4 is provided at angle of 45 degrees with the main optical axis 20 of the VCSEL planar array light source 2 to deflect the VCSEL laser light by 90 degrees, so that a main optical axis 20' of the deflected laser light is perpendicular to the main optical axis 20 of the VCSEL planar array light source 2, providing a reasonable space for VCSEL imaging and coaxial observation. Of course, the planar reflector 4 and the main optical axis 20 of the VCSEL planar array light source 2 may be provided at other angles. At this time, it simply needs to provide the observation position 6 coaxially with the image point along the main optical axis 20' of the deflected laser light.

In order to facilitate real-time observation, between the imaging lens 3 and the virtual image point 2A, the planar reflector 4 is inserted at 45 degrees along the main optical axis, and the planar reflector 4 is a laser reflecting lens (reflecting only the laser light and transmitting visible light), realizing 90 degrees of deflection of laser light path, which in turn enables the real-time coaxial observation behind the planar reflector 4 provided at 45 degrees. There may be a plurality of observation manners, such as direct observation by the naked eyes, observation by the human eyes plus a magnifying glass, imaging by a CCD imaging device, and the like.

In order to improve the reflection effect of the planar reflector 4 on the VCSEL laser light, a highly reflective coating having high reflectivity to the laser light is provided on the surface of the planar reflector 4 facing the imaging lens 3. Since the surface of the planar reflector 4 is coated with the highly reflective coating to ensure the safety of coaxial observation by human eyes, it is safe for coaxial observation by human eyes.

An observation position 6 is provided in the housing 1. The observation position 6 is provided on a side of the planar reflector 4 away from the imaging lens 3 and the optical window 5 (the right side as shown in FIG. 1). The observation position 6 is coaxial with the optical center axis 20' of the deflected VCSEL laser light.

An observation unit, such as a CCD imaging device, is provided at the observation position 6. An image may be transmitted in real time and observed on a display and may also be observed by human eyes through the observation window. Preferably, a magnifying glass 60 is provided at the observation window, and magnification observation of the treatment surface by human eyes can be realized through the magnifying glass 60. The observation of the treatment process and the treatment result is carried out by the observation manners above. In addition, a ring-shaped illumination light source is provided around the observation device for better observation.

For timely heat dissipation of the laser light, the laser above further includes a semiconductor chilling plate (not shown) and a heat pipe 12. A cold end of the semiconductor chilling plate realizes heat conduction and cooling of the optical window 5 through the housing 2, lowering the temperature of the optical window 5. A hot end of the semiconductor chilling plate realizes heat conduction through the heat pipe 12 and the heat sink 11. As a result, the heat energy absorbed by the optical window 5 is transferred out from the heat sink 11 through the semiconductor chilling plate and the heat pipe. In an embodiment of the present invention, the semiconductor chilling plate may be made by utilizing the Peltier effect of a semiconductor material.

A control unit 21 is provided in the laser, and the control unit 21 controls the operation of the semiconductor chilling plate by reading temperature data from a temperature sensor (see below) in a detection unit 22 to achieve a comfortable treatment temperature.

In order to enhance the heat dissipation of the laser light, the laser above further includes a fan 13. The fan 13 is provided below the heat sink 11 for dissipating heat from the heat sink 11. An air inlet and an air outlet are provided on the housing 1 in communication with the area where the fan 13 is located. Heat generated inside the housing 1 is dissipated by the fan 13. The control unit 21 controls the startup and stop of the fan 13.

The semiconductor chilling plate, the VCSEL planar array light source 2 and the fan 13 are all electrically connected to the control unit 21. The control unit 21 may be implemented using a single chip microcomputer, a microcontroller, and the like. The control unit 21 includes a main control panel assembly and a plurality of interfaces, and is electrically connected to the semiconductor chilling plate, the VCSEL planar array light source 2, the fan 13, the detection unit 22, and a control key 23 through the plurality of interfaces.

The laser is provided with the detection unit 22 near the optical window 5. The detection unit 22 includes a skin contact detection element and a temperature detection element. The skin contact detection element is configured to detect whether the optical window 5 (i.e., a treatment head of a laser therapeutic instrument) is in contact with a surface of an object (e.g., a skin surface, i.e., a treatment surface). When the treatment head is not in contact with the skin, no laser light will be emitted by pressing an emit key, and the laser light will be emitted only when the treatment head is in well contact with the skin and the emit key is pressed. The temperature detection element is configured to measure the temperature of the optical window 5 (e.g., sapphire). The skin contact detection element and the temperature detection element above are electrically connected to the control unit 21, respectively.

Moreover, the laser is also provided with the control key 23 connected to the control unit 21 for key control over the laser. In addition, plurality of LED indicators are provided on the housing 1. The plurality of indicators are connected to the control unit 21 for indicating power on and off and tap positions. Specifically, the control keys 23 are classified into a touch key and the emit key. The function of the touch key is touching and holding for three seconds to turn on, touching and holding for three seconds to turn off, and tapping for tap position change. The touch key indicates tap positions by the LED indicators, different tap positions can be applied to different skin color or different parts. The emit key controls the emission of the laser light. The control unit 21 allows the laser light to be emitted only when it detects that an epilator is turned on and is in well contact with the skin, and the emit key is pressed, so as to ensure laser emission safety.

In summary, the laser based on VCSEL imaging and coaxial visualization design provided by the embodiments of the present invention is implemented based on the VCSEL light source and imaging is realized by the imaging lens; and by using the biconvex lens for imaging, the flat-topped spot with excellent uniformity can be obtained at the position of the image point, thereby applying to various types of industrial, medical and scientific research applications. In order to facilitate real-time observation, between the imaging lens and the image point of the undeflected laser light, the laser reflecting lens (reflecting only the laser light and transmitting visible light) is inserted at 45 degrees along the main optical axis. This achieves 90 degrees of laser light path deflection. The coaxial real-time observation can then be carried out behind the reflector at 45 degrees. There may be a plurality of observation manners, such as direct observation by the naked eyes, observation by the human eyes plus a magnifying glass, imaging by a CCD imaging device, and the like.

The laser above can be used as a laser therapeutic instrument, which utilizes the flat-topped spot with excellent uniformity to perform dermatologic surgery treatment, such as hair removal, punctum removal, cauterization of skin lesions, treatment of redness, and the like. The laser above may also be used as an industrial laser to utilize the flat-topped spot with excellent uniformity for plastic keyhole welding, screen bezel welding, screen bezel heating and peptization lamps, and the like.

As described above, the laser based on VCSEL imaging and coaxial visualization design provided by the embodiments of the present invention is not only easy to hold, but also obtains the flat-topped spot with excellent uniformity, a short optical path, and good heat dissipation performance while maintaining small size and light weight; and by providing the magnifying glass, amplification observation is realized while maintaining small size, satisfying the observation requirements of human eyes.

The above provides a detailed description of the laser based on VCSEL imaging and coaxial visualization design provided by the present invention. To a person of ordinary skill in the art, any obvious changes made to the present invention without departing from the essence of the present invention will constitute an infringement of the patent right of the present invention, and shall bear corresponding legal liability.

**Claims**

1. A laser based on VCSEL imaging and coaxial visualization design, comprising

    a housing, defining a hollow holding cavity;

a VCSEL planar array light source and a heat sink, provided in the housing, the VCSEL planar array light source being provided on the heat sink for emitting a VCSEL laser light;

an imaging lens, provided in the housing, the imaging lens being provided coaxially with a main optical axis of the VCSEL planar array light source for imaging the VCSEL laser light, and a distance between the VCSEL planar array light source and the imaging lens being greater than a focal length of the imaging lens;

a planar reflector, provided in the housing, the planar reflector being located behind the imaging lens and provided at an angle of $\alpha$ with the main optical axis of the VCSEL planar array light source, so as to cause deflection of the laser light passing through the imaging lens and to form a real image in a main optical axis of the deflected VCSEL laser light, with $0° < \alpha < 90°$, and the planar reflector being configured to provide total reflection of the VCSEL laser light and to allow transmission of other wavelengths of lights;

an optical window, provided on the housing and at a position of a real image point of the deflected VCSEL laser light; and

an observation position, provided on a side of the planar reflector away from the imaging lens and the optical window, the observation position being provided coaxially with an optical center axis of the deflected VCSEL laser light.

2. The laser according to claim 1, wherein

a distance c between the VCSEL planar array light source and the imaging lens, a distance a between the imaging lens and the planar reflector, and a distance b between the planar reflector and the optical window satisfy the following equation: $1/(a + b) + 1/c = 1/f$, wherein

f represents the focal length of the imaging lens.

3. The laser according to claim 2, wherein

a diameter D of the imaging lens satisfies the following equation: $D \geq m + 2c \times \tan(\theta/2)$, wherein

m is a spot diameter of the VCSEL planar array light source and $\theta$ is a divergence full angle of the VCSEL planar array light source.

4. The laser according to claim 1, wherein
the imaging lens is a biconvex lens.

5. The laser according to claim 1, wherein
a surface of the planar reflector facing the imaging lens is provided with a highly reflective coating for reflecting the VCSEL laser light.

6. The laser according to claim 1, wherein
the planar reflector is provided at an angle of 45 degrees with the main optical axis of the VCSEL planar array light source.

7. The laser according to claim 1, wherein a CCD imaging device is provided at the observation position.

8. The laser according to claim 1, wherein an observation window is provided at the observation position, or a combination of an observation window and a magnifying glass is provided at the observation position.

9. The laser according to claim 1, further comprising a semiconductor chilling plate and a heat pipe, wherein a cold end of the semiconductor chilling plate is configured to cool the optical window, and a hot end of the semiconductor chilling plate is configured to dissipate heat through the heat pipe and the heat sink.

10. The laser according to claim 9, further comprising a fan and a control unit, the fan being provided below the heat sink for dissipating heat from the heat sink, and the semiconductor chilling plate, the VCSEL planar array light source and the fan being electrically connected to the control unit, respectively.

FIG. 1

FIG. 2

FIG. 3

FIG. 4

<div style="text-align:center">**INTERNATIONAL SEARCH REPORT**</div>

| International application No. |
| --- |
| **PCT/CN2022/072597** |

| A. | CLASSIFICATION OF SUBJECT MATTER |
| --- | --- |

H01S 5/183(2006.01)i;  H01S 5/022(2021.01)i;  H01S 5/024(2006.01)i

According to International Patent Classification (IPC) or to both national classification and IPC

| B. | FIELDS SEARCHED |
| --- | --- |

Minimum documentation searched (classification system followed by classification symbols)

H01S5

Documentation searched other than minimum documentation to the extent that such documents are included in the fields searched

Electronic data base consulted during the international search (name of data base and, where practicable, search terms used)

CNABS; CNTXT; VEN; WOTXT; EPTXT; USTXT; CNKI; 百度学术; Web of Science: 同轴, 共轴, 观察, 观测, 摄像, 成像, 反射, 焦距, 实像, coaxial, observ+, image, imaging, ccd, reflect+, focal, length, real

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| PX | CN 112382928 A (BEIJING LASERCONN OPTOELECTRONICS TECHNOLOGY CO., LTD.) 19 February 2021 (2021-02-19) <br> claims 1-10 | 1-10 |
| Y | CN 104339080 A (SCANSONIC MI GMBH) 11 February 2015 (2015-02-11) <br> description, paragraphs [0050]-[0071], and figures 1-8 | 1-10 |
| Y | CN 102827768 A (HUAZHONG UNIVERSITY OF SCIENCE AND TECHNOLOGY) 19 December 2012 (2012-12-19) <br> description, paragraphs [0026]-[0039], and figures 1 and 2 | 1-10 |
| A | CN 109664017 A (XI'AN INSTITUTE OF OPTICS AND PRECISION MECHANICSOF CAS et al.) 23 April 2019 (2019-04-23) <br> entire document | 1-10 |
| A | CN 206065662 U (WUHAN HUAGONG LASER ENGINEERING CO., LTD.) 05 April 2017 (2017-04-05) <br> entire document | 1-10 |

☐ Further documents are listed in the continuation of Box C.    ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **05 March 2022** | **01 April 2022** |

| Name and mailing address of the ISA/CN | Authorized officer |
| --- | --- |
| **China National Intellectual Property Administration (ISA/CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

### INTERNATIONAL SEARCH REPORT
Information on patent family members

International application No.

**PCT/CN2022/072597**

| Patent document cited in search report | | | Publication date (day/month/year) | Patent family member(s) | | | Publication date (day/month/year) |
|---|---|---|---|---|---|---|---|
| CN | 112382928 | A | 19 February 2021 | None | | | |
| CN | 104339080 | A | 11 February 2015 | DE | 102013112244 | A1 | 12 February 2015 |
| | | | | DE | 102013112244 | B4 | 16 February 2017 |
| | | | | CN | 104339080 | B | 24 August 2018 |
| CN | 102827768 | A | 19 December 2012 | CN | 102827768 | B | 25 December 2013 |
| CN | 109664017 | A | 23 April 2019 | None | | | |
| CN | 206065662 | U | 05 April 2017 | None | | | |

Form PCT/ISA/210 (patent family annex) (January 2015)